# EUROPEAN PATENT SPECIFICATION

(11) **EP 3 701 891 B1**
(45) Date of publication and mention of the grant of the patent: **02.08.2023**
(21) Application number: 18871730.0
(22) Date of filing: 22.10.2018
(51) Int. Cl.: A61B 17/64, A61B 17/66, A61B 90/00, G01D 21/02, A61B 17/00, A61B 17/88

(54) **ACTIVE TRACTION DEVICE FOR ORTHOPEDIC SURGERY**
AKTIVE TRAKTIONSVORRICHTUNG FÜR ORTHOPÄDISCHE EINGRIFFE
DISPOSITIF DE TRACTION ACTIVE POUR CHIRURGIE ORTHOPÉDIQUE

(30) Priority: 24.10.2017 KR 20170138302
(43) Date of publication of application: 02.09.2020
(73) Proprietor: Kyungpook National University Industry-Academic Cooperation Foundation, Daegu 41566 (KR)
(72) Inventor: PARK, Ilhyung, Daegu 42016 (KR); JOUNG, Sanghyun, Daegu 41543 (KR); PARK, Chulwoo, Daegu 42195 (KR); LEE, Hyunwoo, Daegu 41185 (KR); PARK, Youngkyun, Daegu 41201 (KR)
(74) Representative: Jordan, Volker Otto Wilhelm
(86) International application number: PCT/KR2018/012491
(87) International publication number: WO 2019/083237

(56) References cited:
- EP-A1- 2 436 324
- CN-B- 103 750 888
- KR-A- 20130 140 559
- KR-B1- 101 433 242
- KR-B1- 101 564 717
- KR-B1- 101 578 489
- US-A1- 2006 276 786
- US-A1- 2008 051 779
- US-A1- 2011 196 371
- US-A1- 2017 007 334

## Description

### [Technical Field]

This disclosure relates to an active retractor and a control method thereof, and more particularly, to an active retractor implemented to detect a stress state of a fractured area during fracture reposition and compensate for a required retracting force, and a control method thereof.

### [Background Art]

The use of metal plates or metal tablets to fix fractured bones is commonplace. The metal plate is used by a surgeon to stabilize, correct or align the bone of a patient. The metal plate is typically fastened to bone fragments by a plurality of fastening units such as screws installed through holes formed in the plate.

For most iliac fractures, the fractured bone fragments are contracted by the muscles, and bone fragments located far away must be properly retracted to align the bone fragments to their proper positions. After being retracting the bone fragments to the original positions, fracture reposition is performed by finely manipulating the bone fragments and fixed using a metal plate or a metal nail.

Thus, there is a need for a tool that may be used to facilitate alignment and subsequent recombination of the fractured bone by applying a retracting force or a compression force thereto.

For the retraction, a conventional retractor just retracts bone fragments by an attractive force or a mechanical configuration and then fix the bone fragments. However, in this method, the retraction is provided only at a fixed location against a stress change of the fractured area that is generated during a fracture reposition process, so there is a limit in providing a retracting force necessary for the intention of a surgical operator.

### [Prior Literature]

### [Patent Literature]

(Patent Literature 0001) Korean Utility Model Registration No. 20-0216141
(Patent Literature 0002) Korean Patent No. 10-1298059
EP 2 436 324 A1 shows an active retractor according to the preamble of claim 1.

### [Disclosure]

### [Technical Problem]

This disclosure is directed to providing an active retractor, which includes a sensor for a stress state of a fractured area, a controller and an actuator to provide a constant retracting force required for a fractured area in real time, and a control method thereof.

The technical problem of the present disclosure is not limited to the technical problem mentioned above, and other technical problems not mentioned will be clearly understood by those skilled in the art from the following description.

### [Technical Solution]

The invention provides an active retractor according to claim 1.

In an embodiment, the measuring device may include: a retracting force measuring sensor connected and installed to the retracting shaft to measure a tensile force or a compressive force of the retracting shaft and then generate tensile force information or compressive force information corresponding thereto to be transmitted to the controller; and a displacement measuring sensor connected and installed to the retracting shaft to measure a moved location of the retracting shaft and generate location information corresponding thereto to be transmitted to the controller.

In an embodiment, the controller may include: a user interface unit configured to display number information and graphic information required for manipulation and receive the retracting request signal from the user; and a driving control unit configured to generate the driving control signal, which is required for the driver to supply a power, by comparing the retracting request signal received from the user interface unit with the measured value transmitted from the measuring device and transmit the driving control signal to the driver.

In an embodiment, the user interface unit may receive a retracting force, a retracting speed, a retracting location, a retracting start/stop, an emergency stop, or postural adjustment of a retracting instrument.

In an embodiment, the user interface unit may receive from the user a retracting distance mode in which a bone fragment is retracted to a retracting location set by the user and then the retracted bone fragment is fixed or a retracting force mode in which a bone fragment is retracted while constantly maintaining a stress of the bone fragment to a retracting force set by the user.

In an embodiment, the driving control unit may generate retracting force information corresponding to the tensile force information or the compressive force information transmitted from the retracting force measuring sensor and transmit the retracting force information to the user interface unit.

In an embodiment, the driving control unit may generate current location information or speed information of the retracting shaft corresponding to the location information transmitted from the displacement measuring sensor and transmit the current location information or speed information to the user interface unit.

In an embodiment, the driving control signal may be a control signal about a retracting speed, a retracting location or a retracting force of the retracting shaft in a digital or analog form.

In an embodiment, the active retractor may further comprise a foothold switch or a remote controller located within a work area of the user to receive a retracting request signal or an operation mode from the user and transmit the retracting request signal or the operation mode to the controller.

In an embodiment, the active retractor may further comprise a location display device formed at one side of the actuator to display a driving location of the retracting shaft in real time.

In an embodiment, the active retractor may further comprise a laser guide formed at one side of the actuator to guide a retracting direction and an aligning direction of a fractured area of a patient.

### [Advantageous Effects]

According to the present invention, the active retractor provides a constant retracting force required for a fractured area in real time.

### [Description of Drawings]

FIG. 1 is a diagram showing a schematic configuration of an active retractor according to an embodiment of the present disclosure.
FIG. 2 shows the active retractor according to an embodiment of the present disclosure in use.
FIG. 3 is a control block diagram for illustrating the controller depicted in FIG. 1.
FIG. 4 is a diagram for illustrating a retracting distance mode.
FIG. 5 is a diagram showing a schematic configuration of an active retractor according to an embodiment of the present disclosure.
FIG. 6 is a diagram showing another embodiment of the active retractor depicted in FIG. 1.
FIG. 7 is a diagram showing a schematic configuration of an active retractor according to an embodiment of the present disclosure.
FIG. 8 is a diagram for illustrating a retracting arm depicted in FIG. 7.
FIG. 9 is a diagram for illustrating an operation method of the retracting arm depicted in FIG. 7.
FIG. 10 is a diagram for illustrating a relaxation damper depicted in FIG. 8.
FIG. 11 is a diagram for illustrating a load limiter depicted in FIG. 8.
FIG. 12 is a diagram for illustrating a housing depicted in FIG. 11.
FIG. 13 is a diagram for illustrating an operation method of the load limiter depicted in FIG. 8.
FIG. 14 is a diagram for illustrating a load adjusting unit depicted in FIG. 11.
FIGS. 15 and 16 are flowcharts for illustrating an active retractor control method according to an embodiment of the present disclosure.
FIG. 17 is a flowchart for illustrating a driving step depicted in FIG. 15.
FIG. 18 is a flowchart for illustrating an active retractor control method according to an embodiment of the present disclosure.
FIG. 19 is a flowchart for illustrating a surgical operation step in which bone fragment tissues are moved by a surgical operator to perform fracture reposition.
FIG. 20 is a flowchart for illustrating a case where the control mode of FIG. 19 is a retracting force mode.
FIGS. 21 to 23 are diagrams for illustrating an algorithm according to each control mode of the retractor.

### [Best Mode]

The following detailed description of the present disclosure refers to the accompanying drawings that show, by way of illustration, specific embodiments in which the present disclosure may be practiced. These embodiments are fully described in detail to enable those skilled in the art to implement the present disclosure. It should be understood that the various embodiments of the present disclosure are different but need not be exclusive to each other. For example, certain shapes, structures and characteristics described herein may be implemented in other embodiments without departing from the scope of the present disclosure in connection with one embodiment. In addition, it is to be understood that the location or arrangement of individual components disclosed in each embodiment may be changed without departing from the scope of the present disclosure. Accordingly, the following detailed description is not to be taken in a limiting sense, and the scope of the present disclosure, if properly described, is defined only by the appended claims along with the full range of equivalents to which such claims are entitled. Like reference numerals in the drawings designate the same or similar functions throughout the several drawings.

Hereinafter, preferred embodiments of the present disclosure will be described in detail with reference to the drawings.

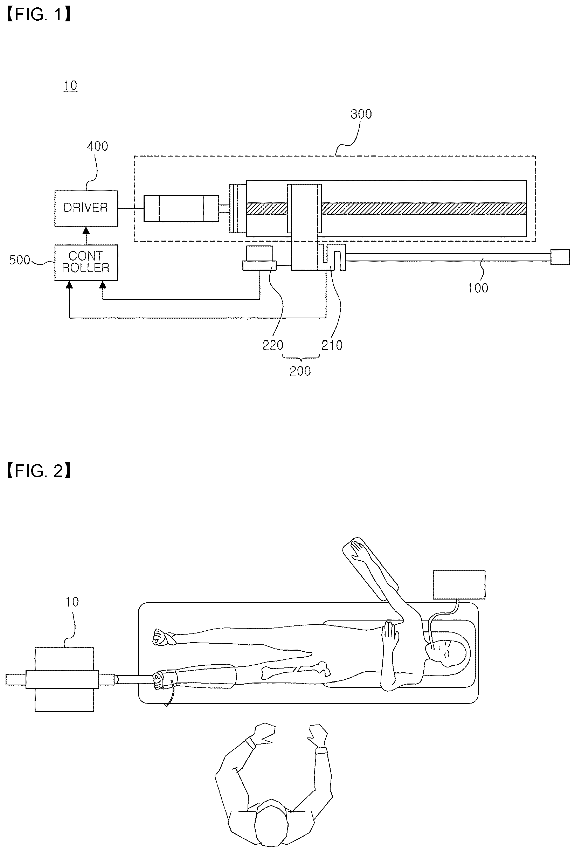

FIG. 1 is a diagram showing a schematic configuration of an active retractor according to an embodiment of the present disclosure.

Specifically, an active retractor 10 according to an embodiment of the present disclosure includes a retracting shaft 100, a measuring device 200, an actuator 300, a driver 400, and a controller 500.

The retracting shaft 100 is connected and installed to the actuator 300 and is fastened to a fractured bone fragment of a patient and retracts the fractured bone fragment in a front and rear direction.

In an embodiment, the retracting shaft 100 may include a gripping means provided at one side terminal thereof to grip a bone fragment of the patient.

The measuring device 200 is connected and installed to the retracting shaft 100 to measure a retracting location or a retracting force of the retracting shaft 100.

In an embodiment, the measuring device 200 includes a retracting force measuring sensor 210 and a displacement measuring sensor 220.

The retracting force measuring sensor 210 is directly connected and installed to the retracting shaft 100 and measures a tensile force or a compressive force of the retracting shaft 100 and generates corresponding tensile force information or compressive force information corresponding thereto. Also, the retracting force measuring sensor 210 transmits the corresponding tensile force information or the corresponding compressive force information to the controller 500.

In an embodiment, as a sensor that may be applied as the retracting force measuring sensor 210, a load cell or a pressure sensor may be used directly. Also, the retracting force measuring sensor 210 may use a principle of measuring displacement generated by an acting force.

The displacement measuring sensor 220 is directly connected and installed to the retracting shaft 100, measures a moved location of the retracting shaft 100, generates location information corresponding thereto, and transmits the generated location information to the controller 500.

In an embodiment, as a sensor that may be applied as the displacement measuring sensor 220, an optical/magnetic absolute encoder, an optical/magnetic incremental encoder, or a photo interrupt or a hall switch may be applied to a required position.

The actuator 300 is connected and installed to the retracting shaft 100, receives a power from the driver 400, and drives the retracting shaft 100 in the front and rear direction in response to the supplied power.

In an embodiment, the actuator 300 corresponds to a driving unit that generates a linear movement and a retracting force for moving a bone fragment to a location necessary for retracting, and may include a reducer capable of adjusting speed and torque according to an electric control of the driver 400 and a ball screw for converting rotational movement of the reducer into linear movement.

The driver 400 receives a driving control signal from the controller 500 and supplies a power required for driving the actuator 300 to the actuator 300 according to the received driving control signal.

In an embodiment, the driver 400 supplies a power required for driving the actuator 300 to the actuator 300 according to the control signal of the controller 500, and the driver 400 may be a three-phase inverter including a speed and torque controller or a power switch in the form of a pulse width modulation (PWM) control.

The controller 500 compares the retracting request signal input from the user with a measured value transmitted from the measuring device 200, generates a driving control signal, which is required for the driver 400 to supply a power, corresponding to the compared value, and transmits the corresponding driving control signal to the driver 400.

In an embodiment, the controller 500 may output the control signal required for the driver 400 by comparing the retracting request signal set by the user to follow the retracting force, the retracting location and the retracting speed with the measured values of the retracting force measuring sensor 210 and the displacement measuring sensor 220.

In an embodiment, the controller 500 may calculate information about a stress of the fractured area measured by the retracting force measuring sensor 210 and calculate a speed by differentiating the measured position with respect to the current position and time of the retracting shaft 100 measured by the displacement measuring sensor 220. Also, the controller 500 may compare the information calculated by each sensor with user setting formation and transmit a driving control signal to the driver 400 to maintain the necessary retracting force and the necessary retracting location. Here, the driving control signal transmitted to the driver 400 is a control signal about speed, location and retracting force and may be in a digital or analog form.

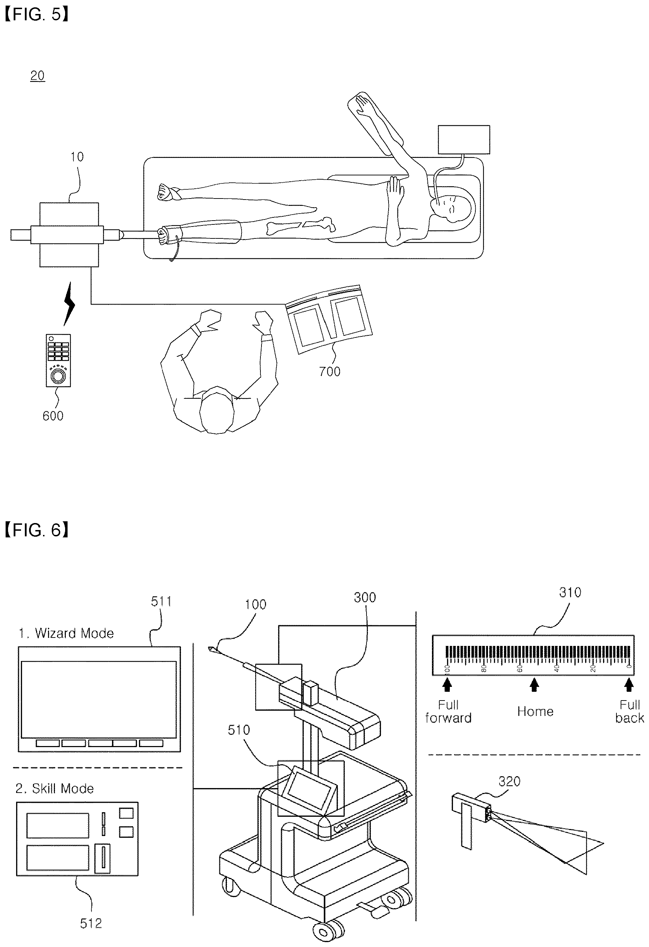

The active retractor 10 configured as above may further include a display device 310 and a laser guide 320 (see FIG. 6).

The display device 310 is formed at one side of the actuator 300, and displays a driving location of the retracting shaft 100 in real time or a maximum contraction/relaxation state of a pulling rod of the retracting shaft 100. In this case, the display device 310 displays the maximum extension state of the retracting shaft 100 as "Full forward", displays the maximum contraction state as "Full back", and divides the state into a plurality of stages so that the user may visually recognize the retracting state of the retracting shaft 100 in an easy way.

The laser guide 320 is formed at one side of the actuator 300 and may irradiate a guide laser to guide a retracting direction and an alignment direction of the fractured area of the patient.

The active retractor 10 configured as above includes a measuring device 200 capable of detecting a stress state of the fractured area during fracture reposition, a controller 500 and an actuator 300, thereby providing a constant retracting force necessary for the fractured area in real time.

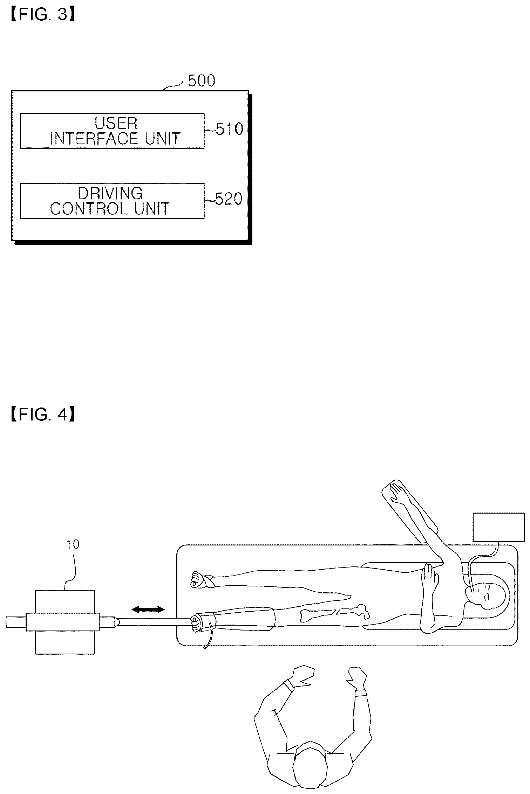

FIG. 3 is a control block diagram for illustrating the controller depicted in FIG. 1.

Referring to 3, the controller 500 includes a user interface unit 510 and a driving control unit 520.

The user interface unit 510 displays number information and graphic information necessary for manipulation, receives a retracting request signal from the user, and transmits the received retracting request signal to the driving control unit 520.

Referring to FIG. 6, the user interface unit 510 may implement a user interface in consideration of the retractor characteristics and fracture reposition procedure. First, 1) in a wizard mode, the user interface unit 510 implements an environment that enables the user to perform safe manipulation without knowing the manual and provides a step-by-step method in which the setting and check items for each step are displayed and confirmed by the user, and 2) in a skill mode 512, the user interface unit 510 provides only a basic panel that displays various setting values and a state of the driver to support a rapid use environment for experienced users.

In an embodiment, the user interface unit 510 is a device for displaying numerical information and graphic information necessary for retractor operation, and may utilize a GUI program based on a mechanical electrical contact or a touch screen. A main user display (output) function of the user interface unit 510 may display a retracting force, a retracting speed and a retracting location currently measured as well as various alarms such as a retracting upper limit, a retracting lower limit and a retracting-executing mark. Also, a main user input function of the user interface unit 510 may support a manipulation panel for retracting force setting input, retracting speed setting input, retracting location setting input, retracting start/stop, emergency stop, and retracting instrument postural adjustment.

In an embodiment, the user interface unit 510 may have a simple display form in which necessary setting inputs and displays are listed on the screen for user convenience and safe use, or a manual (wizard) display form in which need-to-know items or medical information important for the retractor operation of the user are displayed sequentially suitable for the surgical operation procedure so as to be checked by the user.

In an embodiment, the user interface unit 510 is a hardware interface for user convenience. In order to improve visibility and operation inconvenience caused by a long distance from a manipulating panel (the user interface) of the retractor or a deviation from a work area of the user, a foothold switch 700 and a remote controller 600, explained later with reference to FIG. 5, may be provided in the work area of the user.

The driving control unit 520 receives the retracting request signal from the user interface unit 510, receives the measured value from the measuring device 200, compares the received retracting request signal with the measured value, and generates a driving control signal required for power supply to the driver 400 corresponding value to the compared value, and transmits the corresponding generated driving control signal to the driver 400.

In an embodiment, the user interface unit 510 may receive a retracting force, a retracting speed, a retracting location, a retracting start/stop, an emergency stop, or postural adjustment of the retracting instrument.

In an embodiment, the user interface unit 510 may receive from the user a retracting distance mode in which a bone fragment is retracted to a retracting location set by the user and then the retracted bone fragment is fixed, or a retracting force mode in which a bone fragment is retracted while maintaining the stress of the bone fragment to a retracting force set by the user.

At this time, the retracting distance mode may be utilized when a bone fragment is always fixed at a constant position after fracture reposition is completed since the bone fragment is moved and then the retraction is fixed. In the retracting force mode, retraction is performed while maintaining the stress of the bone fragment as much as the retracting force set by the user, so the retracting force mode may be utilized when stable relaxation minimizing muscle tissue damage in an over-rap state caused by muscle contraction on the fractured area is required before fracture reposition or when the fracture reposition is required. In particular, when the fracture reposition is performed, the retractor compensates for the stress of the fractured area on the influence of the attraction caused by the invention of a surgical operator, thereby reducing the burden on the surgical operator and enabling a stable fracture reposition procedure.

For example, referring to FIG. 4, the foot of the patient is retracted by a preset distance (for example, 5 cm to 20 cm) and fixed according to the present disclosure, which allows the surgical operator to avoid unnecessary forces and to make precise retraction, thereby ensuring the accuracy and stability of the surgical operation.

In an embodiment, the driving control unit 520 may generate retracting force information corresponding to the tensile force information or the compressive force information transmitted from the retracting force measuring sensor 210, and transmit the retracting force information to the user interface unit 510.

In an embodiment, the driving control unit 520 may generate current location information or speed information of the retracting shaft 100 corresponding to the location information transmitted from the displacement measuring sensor 220 and transmit the generated information to the user interface unit 510.

In an embodiment, the driving control signal is a control signal for retracting speed, retracting location or retracting force of the retracting shaft 100 and may be a digital or analog signal.

In an embodiment, the driving control unit 520 may calculate the information on the stress of the fractured area measured by the retracting force measuring sensor 210 and transmit the information to the user interface unit 510, and may calculate the speed by differentiating the location measured with respect to the current location and time of the retracting shaft 100 measured by the displacement measuring sensor 220 and transmit the calculated location and speed information to the user interface unit 510.

In an embodiment, the driving control unit 520 may transmit the measured values measured by the retracting force measuring sensor 210 and the displacement measuring sensor 220 to the user interface unit 510 to be displayed on the user interface unit 510.

FIG. 5 is a diagram showing a schematic configuration of an active retractor according to an embodiment of the present disclosure.

Specifically, the active retractor 20 according to an embodiment of the present disclosure includes a retracting shaft 100, a measuring device 200, an actuator 300, a driver 400, a controller 500, a foothold switch 700, and a remote controller 600. Here, the retracting shaft 100, the measuring device 200, the actuator 300, the driver 400 and the controller 500 are the same as the components of shown in FIG. 1 and thus will not described in detail here.

The remote controller 600 is located within the work area of the user, and receives the retracting request signal (for example, a request signal for changing the location of the retracting shaft 100 or increasing or decreasing the retracting force of the retracting shaft 100) or an operation mode from the user and transmits the corresponding retracting request signal to the controller 500 through wired or wireless communication.

The foothold switch 700 is located within the work area of the user. If the user is not able to use both hands for reasons such as surgery, the foothold switch 700 receives a retracting request signal (for example, a request signal for changing the location of the retracting shaft 100 or increasing or decreasing the retracting force of the retracting shaft 100) or an operation mode and transmits the same to the controller 500.

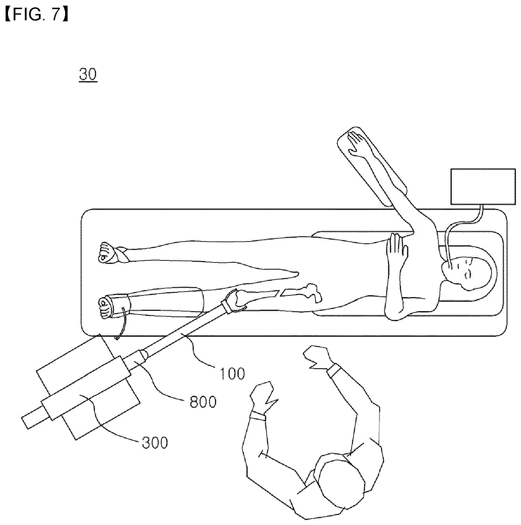

FIG. 7 is a diagram showing a schematic configuration of an active retractor according to an embodiment of the present disclosure.

Specifically, the active retractor 30 according to an embodiment of the present disclosure includes a retracting shaft 100, a measuring device 200, an actuator 300, a driver 400, a controller 500, and a retracting arm 800. Here, the retracting shaft 100, the measuring device 200, the actuator 300, the driver 400 and the controller 500 are the same as the components of FIG. 1 and thus will not be described in detail here.

The retracting arm 800 is mounted and installed to the actuator 300 and is driven in the front and rear direction corresponding to the driving of the actuator 300. Also, one side of the retracting shaft 100 is inserted into the retracting arm 800 and fastened thereto so that the retracting shaft 100 is driven in the front and rear direction, and the fastening is released if a force greater than a preset force is applied thereto, to relax the retracting shaft 100.

The active retractor 30 configured as above implements a double safety device as a stabilizer of a mechanical configuration independent from an electrical device, and it is possible to prevent a safety accident that an excessive force is transferred to an affected part during the retracting process.

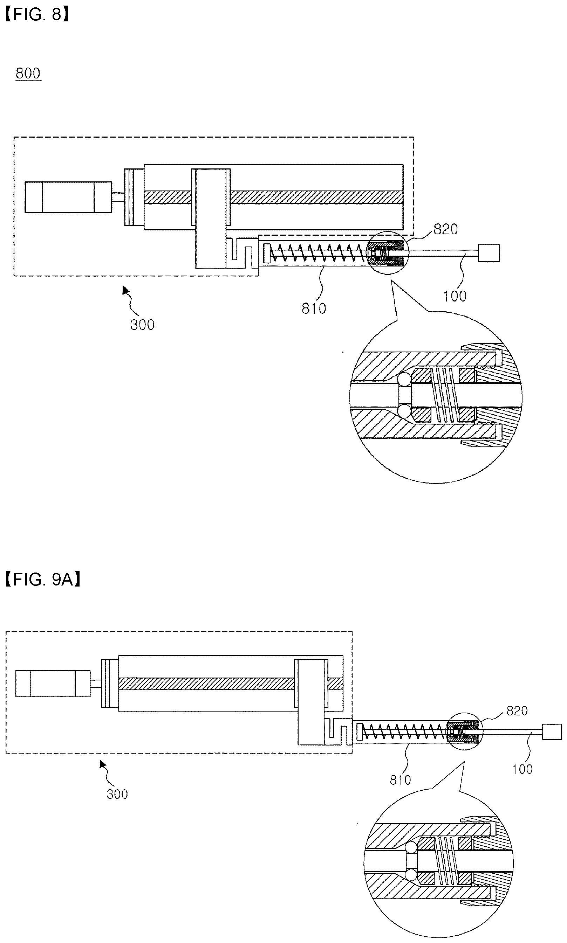

FIG. 8 is a diagram for illustrating a retracting arm depicted in FIG. 7.

Referring to FIG. 8, the retracting arm 800 includes a relaxation damper 810 and a load limiter 820.

The relaxation damper 810 is mounted and installed to the actuator 300, and one side of the retracting shaft 100 is inserted into an insert groove formed therein. Also, when the retracting shaft 100 is released and relaxed from the insert groove, the relaxation damper 810 absorbs a part of the transferred force.

The load limiter 820 is mounted and installed to an open one side of the relaxation damper 810 and fastens the retracting shaft 100. When a force equal to or greater than a preset force is supplied thereto from the actuator 300, the load limiter 820 releases the fastening.

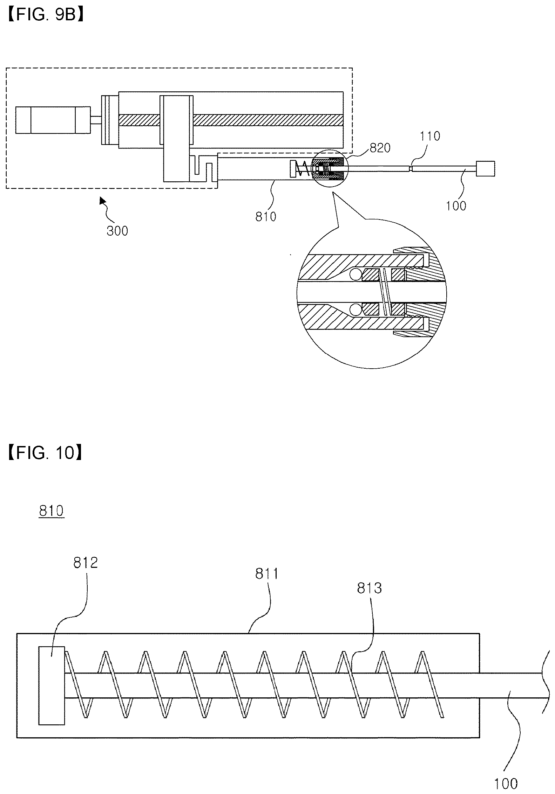

In a normal state in which the retracting arm 800 configured as above retracts the affected part with a suitable force as shown in FIG. 9(A), the actuator 300 and the retracting arm 800 serving as a safety device are moved together to transmit the retracting force of the actuator 300. However, in an abnormal state in which an excessive force is applied to damage the affected part, as shown in FIG. 9(B), the load limiter 820 fastened to the retracting shaft 100 releases the fastening to separate the retracting shaft 100 from the relaxation damper 810, and the relaxation damper 810 may slowly perform a relaxation operation with a maximum stroke (a maximum stroke of the relaxation damper 810) by itself.

FIG. 10 is a diagram for illustrating a relaxation damper depicted in FIG. 8.

Referring to FIG. 10, the relaxation damper 810 includes a piston body 811, a piston unit 812, and a relaxation elastic unit 813.

The piston body 811 has an insert groove formed therein so that one side of the retracting shaft 100 is inserted therein, and the piston body 811 is connected and installed to the actuator 300.

The piston unit 812 is mounted and installed to one end of the retracting shaft 100 and slides in the front and rear direction in the insert groove of the piston body 811.

The relaxation elastic unit 813 is inserted between the insert groove of the piston body 811 and the piston unit 812 to press the piston unit 812 in one direction.

In an embodiment, the relaxation elastic unit 813 may be implemented as a spring, a solid or a fluid.

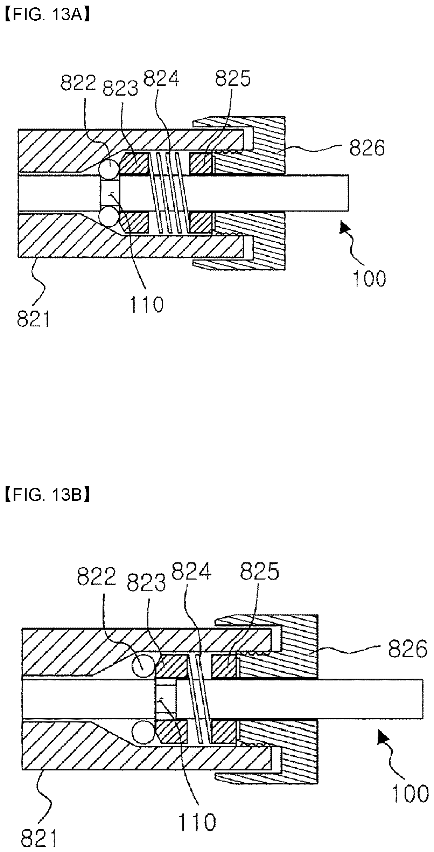

In the relaxation damper 810 configured as above, if the retracting shaft 100 comes into a freely movable state without restraint as shown in FIG. 13(B) as the load limiter 820 is loosened, the piston unit 812 is prevented from suddenly sliding in the front and rear direction in the insert groove of the piston body 811 due to the elastic force of the relaxation elastic unit 813, so the relaxation damper 810 may slowly perform a relaxation operation with a maximum stroke (a maximum stroke of the relaxation damper 810) by itself.

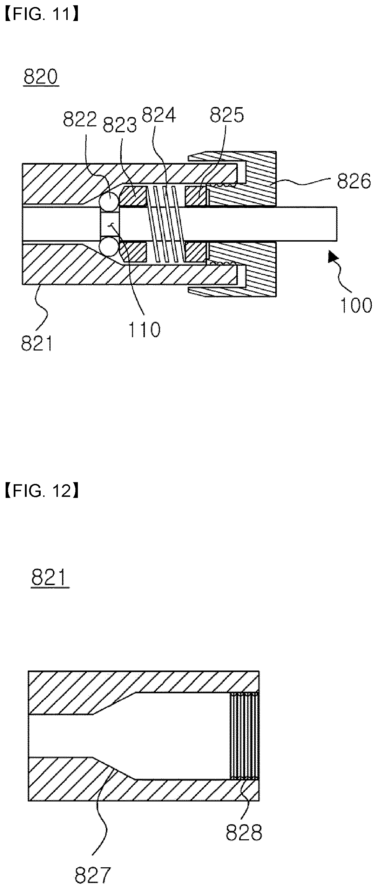

FIG. 11 is a diagram for illustrating a load limiter depicted in FIG. 8.

Referring to FIG. 11, the load limiter 820 includes a housing 821, a plurality of fastening units 822, a pressing unit 823, an elastic unit 824, a supporting unit 825, and a load adjusting unit 826.

The housing 821 is mounted and installed to one side of the relaxation damper 810, so that the plurality of fastening unit 822, the pressing unit 823, the elastic unit 824, the supporting unit 825 and the load adjusting unit 826 are accommodated in a space formed therein.

In an embodiment, the housing 821 may form a funnel-shaped inclined surface 827 at an inner portion thereof (see FIG. 12).

In an embodiment, the housing 821 may have a fastening thread 828 formed at an inlet portion thereof to fasten the insert thread 8263 (see FIG. 12).

The fastening unit 822 is fastened to the fastening groove 110 formed at an outer surface of the retracting shaft 100.

In an embodiment, the fastening unit 822 may be formed in a spherical shape or a wedge shape.

In an embodiment, the fastening unit 822 may move into the housing 821 along the inclined surface 827 to fasten the fastening groove 110 as the distance from another fastening unit 822 decreases.

The pressing unit 823 is connected and installed to the other side of the fastening unit 822 to press the fastening unit 822 into the housing 821.

The elastic unit 824 is connected and installed to the other side of the pressing unit 823 to press the pressing unit 823 into the housing 821 by an elastic force.

The supporting unit 825 is inserted into the other side of the elastic unit 824 to support the elastic unit 824.

The load adjusting unit 826 is mounted and installed to one open side of the housing 821 to adjust the insertion location of the supporting unit 825.

The load limiter 820 configured as above may release the fastening unit 822 when the force supplied from the actuator 300 is greater than the fastening force of the fastening unit 822 that fastens the fastening groove 110.

The load limiter 820 configured as above adjusts the fastening force of the fastening unit 822 placed in the fastening groove 110 of the retracting shaft 100 by the tension of the elastic unit 824, to release the fastening unit 822 when the force applied to the retracting shaft 100 is greater than the tightening force, thereby preventing an excessive force from being transferred to the retracting shaft 100.

In the load limiter 820 configured as above, in a normal state in which a force smaller than the force set by the retracting shaft 100 is transmitted, the fastening of the load limiter 820 is not released from the retracting shaft 100 as shown in FIG. 13(A) and thus may move together with the relaxation damper 810 in the left and right direction. However, in an abnormal state in which a force greater than the force set by the retracting shaft 100 is transmitted, the fastening unit 822 moves along the fastening groove 110 of the retracting shaft 100 and is pushed by the compressive force of the elastic unit 824 to deviate from the fastening groove 110 of the retracting shaft 100, so the fastening unit 822 is released from the retracting shaft 100 as shown in FIG. 13(B) to deviate from the binding force of the fastening unit 822. Thus, the retracting force transmitted through the fastening unit 822 is interrupted and the retracting shaft 100 comes into a freely movable state without restraint.

At this time, if the retracting shaft 100 is in the freely movable state, since a sudden movement may occur due to the external environment, the relaxation damper 810 is provided thereto to prevent the sudden movement.

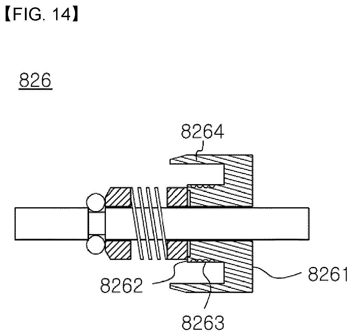

FIG. 14 is a diagram for illustrating a load adjusting unit depicted in FIG. 11.

Referring to FIG 14, the load adjusting unit 826 includes a handle means 8261, an adjustment means 8262 and a cover means 8264.

The handle means 8261 has an adjustment means 8262 formed on one side and allows the user to grip and rotate the handle means 8261 by hand.

The adjustment means 8262 is formed at one side of the handle means 8261 to adjust the insertion location of the supporting unit 825 while being inserted into or separated from the housing 821 in response to the rotation direction of the handle means 8261.

In an embodiment, the adjustment means 8262 may have an insert thread 8263 formed at an outer side so as to be fastened to the housing 821.

The cover means 8264 extends from the handle means 8261 toward the housing 821 to cover the open one side of the housing 821.

In an embodiment, the cover means 8264 may have an uneven portion formed at an outer surface thereof to form a frictional force when being gripped by hand of the user.

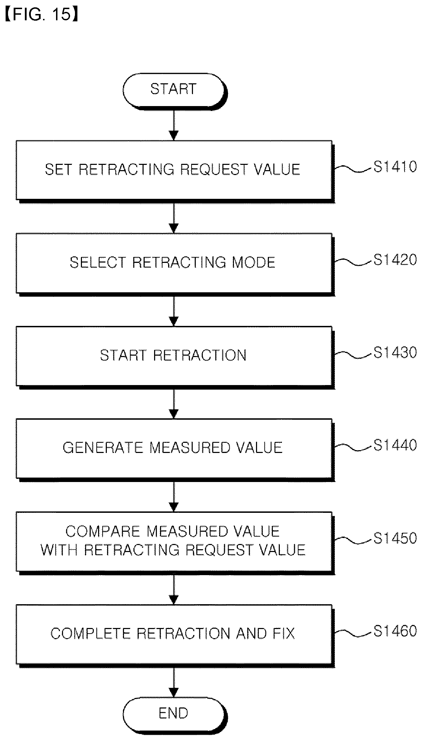

FIG. 15 is a flowchart for illustrating an active retractor control method according to an embodiment of the present disclosure.

Referring to FIG. 15, in an active retractor control method, firstly, the controller 500 receives and sets a retracting request signal from a user (S1410).

If the retracting request signal is input in the step S1410, the controller 500 allows the user to select a retracting mode (S1420).

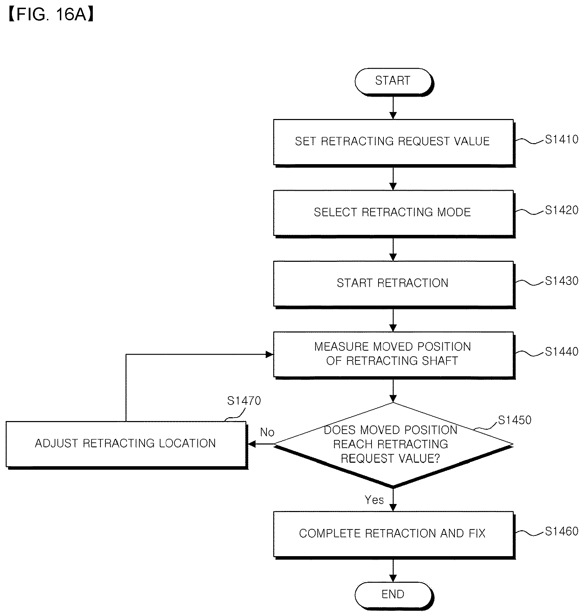

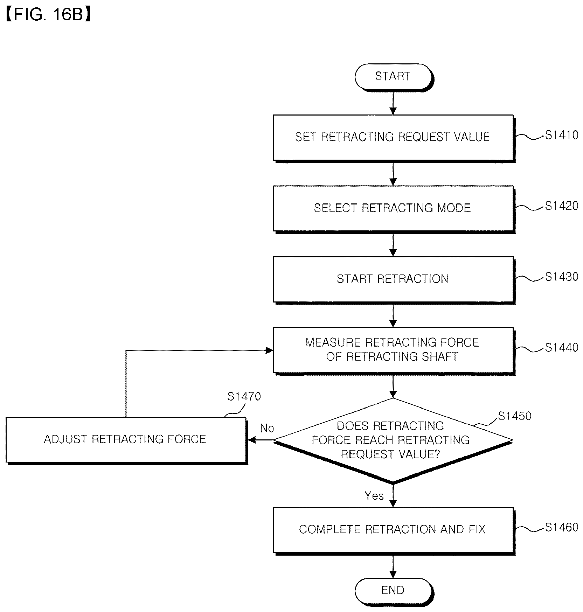

In an embodiment, in the step (S1420) of allowing the user to select a retracting mode, as shown in FIG. 16, the retracting distance mode in which a bone fragment location is moved by the set retracting location and then the retracted bone fragment location is fixed (FIG. 16A, in the case of the "retracting distance mode" in the step S1420) or the retracting force mode in which a bone fragment is retracted while constantly maintaining the stress by the set retracting force (FIG. 16B, in the case of the "retracting force mode" in the step S1420) may be selected by the user.

In an embodiment, the step S1420 of allowing the user to select a retracting mode, while the retraction is being performed in any one of the retracting distance mode and the retracting force mode, if a mode shifting request is received from the user, the retracting mode in progress is temporarily stopped, and then the retraction is performed in the other mode. If the mode is changed from the retracting distance mode to the retracting force mode, the retracting force value measured in the retracting distance mode is set as an initial setting value of the retracting force. However, the initial setting value may be increased or decreased according to an input of the user.

If the retracting mode is selected in the step S1420 described above, the actuator 300 connected and installed to the retracting shaft 100 drives the retracting shaft 100 in the front and rear direction under the control of the controller 500 (S1430).

If the retracting shaft 100 is driven in the step S1430 described above, the measuring device 200 connected and installed to the retracting shaft 100 measures a tensile force, a compressive force or a moved location of the retracting shaft 100 and then generates a measured value corresponding thereto and transmits the measured value to the controller 500 (S1440).

In an embodiment, in the step S1440 described above, as shown in FIG. 16, the displacement measuring sensor 220 measures a moved position of the retracting shaft 100 (FIG. 16A, S1440) or the retracting force measuring sensor 210 measures a retracting force of the retracting shaft 100 (FIG. 16B, S1440).

If the measured value is transmitted in the step S1440 described above, the controller 500 compares the measured value transmitted from the measuring device 200 with the retracting request signal input from the user (S1450).

In an embodiment, in the step S1450 described above, if the retracting location of the retractor 100 does not reach the retracting request signal in the "retracting distance mode" as shown in FIG. 16A (in case of "No" in the step S1450), the step S1470 of readjusting the retracting location is executed as described below. However, if the retracting location of the retractor 100 reaches the retracting request signal (in case of "Yes" in the step S1450), the retraction is completed and the process proceeds to the step S1460 of fixing the retractor 100. In addition, if the retracting force of the retractor 100 does not reach the retracting request signal in the "retracting force mode" as shown in FIG. 16B (in the case of "No" of the step S1450), the step S1470 is executed. However, if the retracting force of the retractor 100 reaches the retracting request signal (in case of "Yes" of the step S1450), the retraction is completed and the process may proceed to the step S1460 of fixing the retractor 100.

When the retracting request signal is compared in the step S1450 described above, if the measured value reaches the retracting request signal, the controller 500 completes the retracting and maintains the retracting shaft 100 in a fixed state (S1460).

The active retractor control method having the steps as described above may further include, after the step S1450 of comparing, a step of adjusting the retracting force (the tensile force or the compressive force) or the moved location of the retracting shaft 100 if the measured value does not reach the retracting request signal (S1470 of FIG. 16).

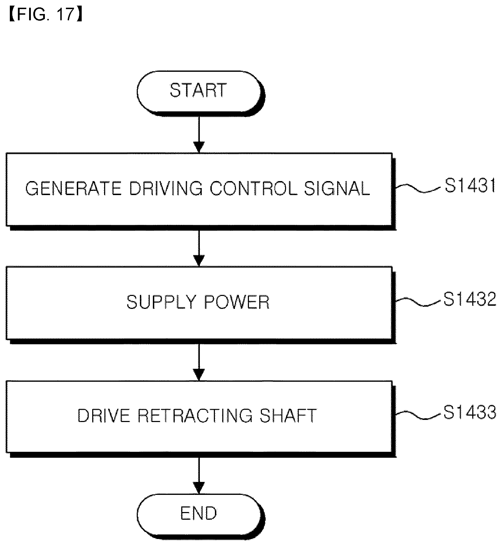

FIG. 17 is a flowchart for illustrating a driving step depicted in FIG. 15.

Referring to FIG. 17, in the step S1430 of driving, firstly, the controller 500 generates a driving control signal required for the driver 400 to supply a power to the actuator 300, and transmits the driving control signal to the driver 400 (S1431).

If the driving control signal is transmitted in the step S1430 described above, the driver 400 supplies a power required for driving the actuator 300 to the actuator 300 according to the driving control signal transmitted from the controller 500 (S1432).

If power is supplied in the step S1432 described above, the actuator 300 drives the retracting shaft 100 in the front and rear direction according to the power supplied from the driver 400 (S1433).

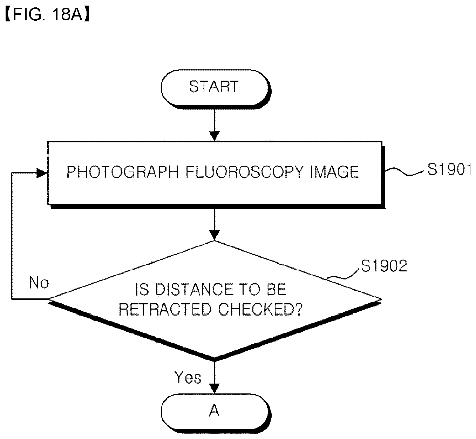

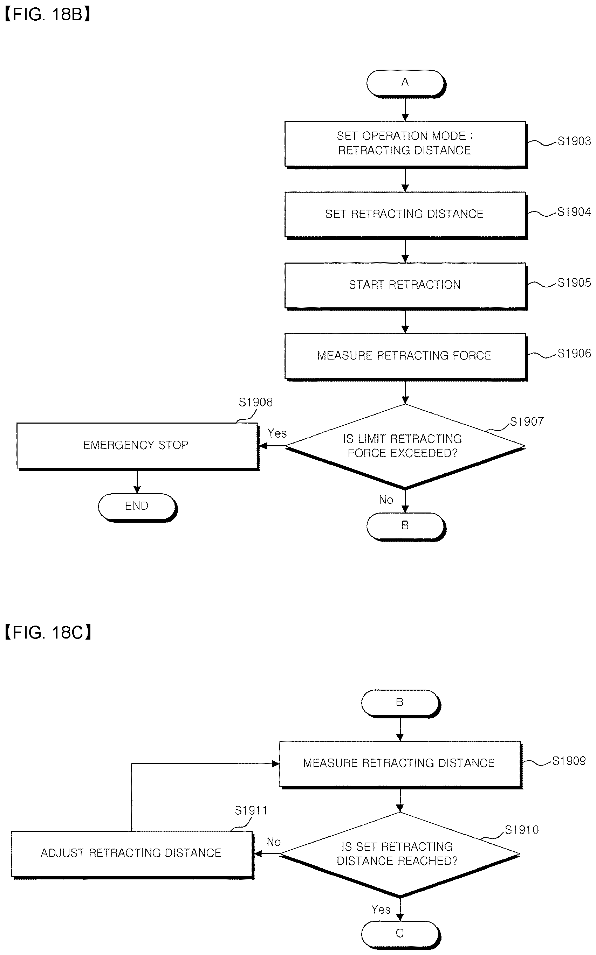

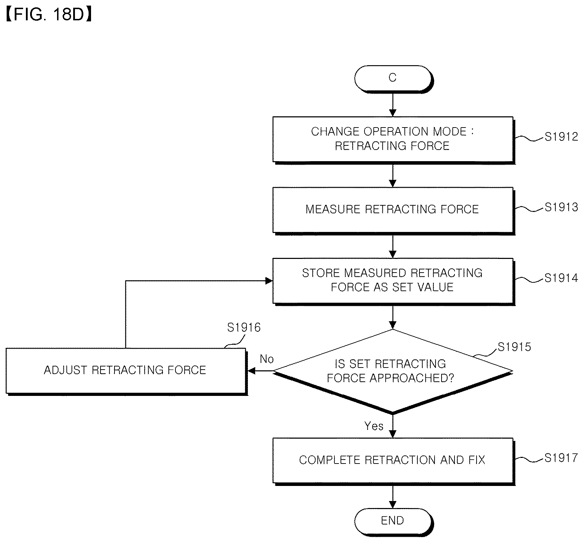

FIG. 18 is a flowchart for illustrating an active retractor control method according to an embodiment of the present disclosure.

Referring to FIG. 18, in the active retractor control method, firstly, a fluoroscopy image of a fractured bone fragments of the patient is taken until the distance to be retracted is checked at a separately provided imaging device (not shown in the drawings for convenience of description) (in case of "Yes" in the step S1902) (S1901).

If the distance to be retracted is checked in the step S1902 described above, a retracting distance mode is input from the user in the controller 500 as a preretracting operation mode (S1903), and the retracting mode is set as the retracting distance mode according to the inputted retracting distance mode (S1904).

The retracting distance mode is set as the pre-retraction operation mode in the step S1904 described above, retraction starts according to the retracting distance mode set in the actuator 300 (S1905), and the retracting force is measured after the retraction starts in the measuring device 200 (S1906). If the measured retracting force exceeds a limit retracting force (received from the user and stored in advance) (in case of "Yes" in the step S1907), the retraction is emergently stopped to complete the retraction, thereby preventing an excessive retracting force from being applied to the affected part of the patient (S 1908).

In an embodiment, if the retraction is emergently stopped to complete the retraction, the controller 500 notifies the user of the emergency stop by a visual and audio device after the retraction stops, and the process may enter a normal mode again after inputting the confirmation of the user.

If the retracting force does not exceed the limit retracting force in the step S1907 described above (in the case of "No" in the step S1907), the actuator 300 performs the retraction according to the initially set retracting distance mode under the control of the controller 500, and the measuring device 200 measures the retracting distance (S1909). After that, if the measured retracting distance does not reach the set retracting distance (in the case of "No" in the step S1910), the controller 500 adjusts the retracting distance (S1911).

If the retracting distance measured in the step S1910 described above reaches the set retracting distance (in the case of "Yes" in the step S1910), the controller 500 changes the retracting mode to the retracting force mode and performs the retraction (S1912), measures the retracting force (S1913), and stores the measured retracting force as a set value (S1914).

If the retracting force stored in the above step S1914 does not approach the retracting request signal input from the user (in the case of "No" in the step S1915), the controller 500 adjusts the retracting force until the retracting force corresponds to the retracting request signal (S1916).

If the retracting force measured in the step S1913 described above corresponds to the retracting request signal (in case of "Yes" of the step S1915), the retraction is completed and fixed (S1917).

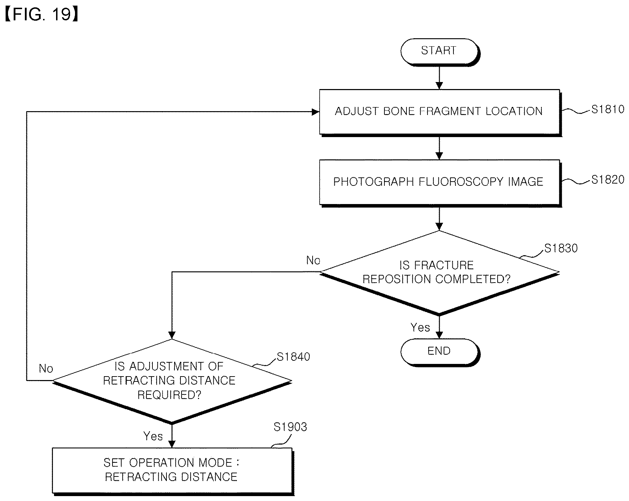

The active retractor control method having the above steps may further include a step in which, as shown in FIG. 19, after the retractor reaches a target retraction setting, a surgical operator directly moves a bone fragment tissue to complete fracture reposition or, if an additional retracting force is needed, the surgical operator capable of using the retractor moves the bone fragment tissue and performs fracture reposition.

In the step of performing fracture reposition by moving the bone fragment tissue by the surgical operator, firstly, the bone fragment location of the patient is adjusted (S1810), and then the fluoroscopy image is photographed (S1820). If the fracture reposition is completed by adjusting the bone fragment in the step S1810 described above, the surgical operation is immediately terminated (in the case of "Yes" in the step S1830) without the retraction procedure as described above.

If the fracture reposition is not completed by adjusting the bone fragment in the step S1830 described above (in the case of "No" in the step S1830), or if the retracting distance adjustment is not necessary through the fluoroscopy image (in the step of "No" in the step S1840), the bone fragment location is adjusted again (S1810).

If the fracture reposition is not completed in the step S1840 described above and it is needed to adjust the retracting distance (in the case of "Yes" in the step S1840), the process comes to the step S1903 so that the retraction operation mode is set.

The step of performing fracture reposition by moving the bone fragment tissue by the surgical operator is a step in which the surgical operator moves the bone fragment directly (by hand) to perform fracture reposition after the retraction is completed using the retractor. At this time, since the bone fragment is moved directly by hand, a pulling force is added to the fractured part to change the retracting force. If the control mode of the retractor is the retracting force mode, the retractor attempts to compensate for the retracting force generated by the pulling and pushing force of the surgical operator (during a bone fragment repositioning process (= fracture reposition)). However, the intention of the surgical operator is reflected on the compensation of the retractor for the retracting force, so the retractor is manipulated by direct teaching.

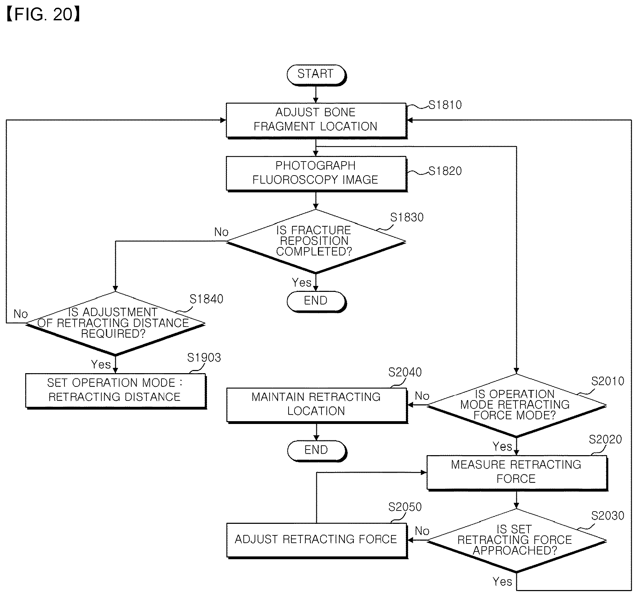

In the step of performing the fracture reposition by moving the bone fragment tissue by the surgical operator as described above, if the control mode of the retractor is maintained in the retracting force mode, a surgical operator/retractor collaboration step as shown in FIG. 20 may be further included.

When the bone fragment location is adjusted in the step S1810 described above, if the operation mode is the retracting force mode ("Yes" in the step S2010), the retracting force is measured (S2020). Also, if the retracting force measured in the step S2020 is close to the set retracting force ("Yes" in the step S2030), the bone fragment location is adjusted again (S1810). If the retracting force measured in the step S2020 does not approach the set retracting force ("No" in the step S2030), the retracting force is adjusted (S2050), and then the retracting force is measured again until approaching the set retracting force.

Finally, a control algorithm used in each retracting mode according to each embodiment of the present disclosure will be described as follows.

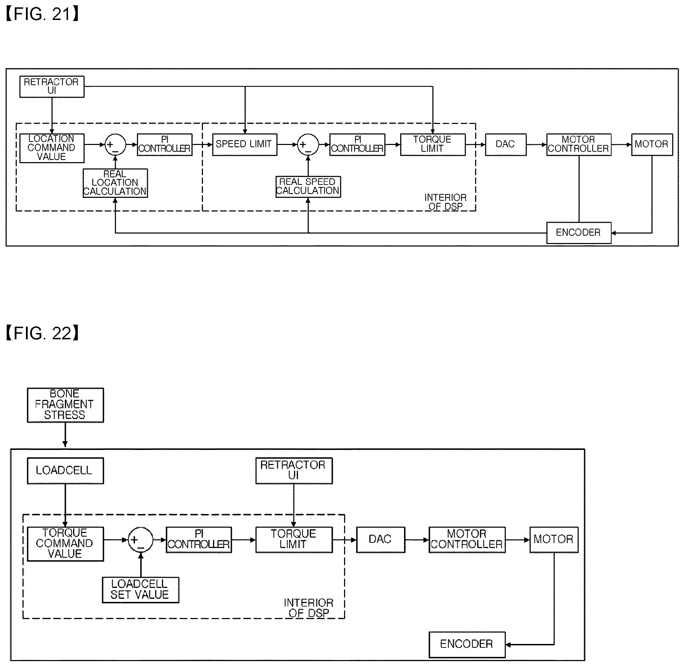

First, FIG. 21 is a diagram for illustrating a control algorithm of the retracting distance mode. In the control algorithm of the retraction distance mode, control is made based on a retracting location (a location command value), a retracting speed (a speed limit), and a retracting force (a torque limit) input by the manipulation of the user in a retractor UI.

Next, FIG. 22 is a diagram for illustrating a control algorithm of the retracting force mode. In the control algorithm of the retracting force mode, after the stress between the fractured bone fragments is measured by the load cell, the measured retracting force becomes a torque control command and is controlled based on the retracting force limit (the torque limit) input by the manipulation of the user in the retractor III.

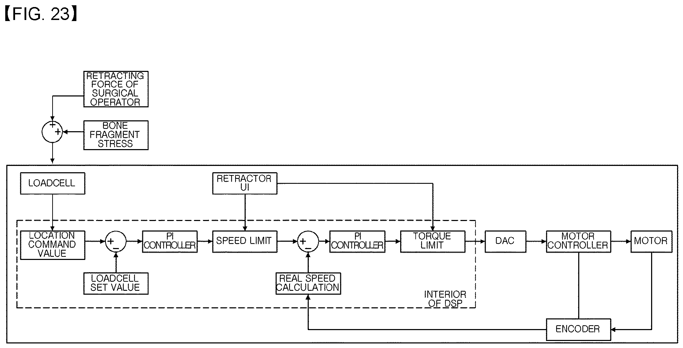

FIG. 23 is a diagram for illustrating a surgical operator/retractor collaborative control algorithm in the surgical operator/retractor collaborating step of FIG. 20. Here, in the surgical operator/retractor collaborative control algorithm, a force obtained by adding the stress between the fractured bone fragments and the attractive force by the fracture reposition of the surgical operator is transferred to the load cell, and the measured retracting force becomes a location control command and is controlled based on the retracting speed (the speed limit) and the retracting force (the torque limit) input by the manipulation of the user in the retractor UI.

Although the present disclosure has been described above with reference to the embodiments, it can be understood that the present disclosure can be variously modified and changed by those skilled in the art without departing from the scope of the present disclosure defined in the appended claims.

**[Reference Symbols]**

| | | | |
|---|---|---|---|
| 100: | retracting shaft | 110: | fastening groove |
| 200: | measuring device | 210: | retracting force measuring sensor |
| 220: | displacement measuring sensor | | |
| 300: | actuator | 310: | location display device |
| 320: | laser guide | 400: | driver |
| 500: | controller | 510: | user interface unit |
| 511: | Wizard Mode | 512: | Skill Mode |
| 520: | driving control unit | 700: | foothold switch |
| 600: | remote controller | 800: | retracting arm |
| 810: | relaxation damper | 811: | piston body |
| 812: | piston unit | 813: | relaxation elastic unit |
| 820: | load limiter | 821: | housing |
| 822: | fastening unit | 823: | pressing unit |
| 824: | elastic unit | 825: | supporting unit |
| 826: | load adjusting unit | 8261: | handle means |
| 8262: | adjustment means | 8263: | insert thread |
| 8264: | cover means | 827: | inclined surface |
| 828: | fastening thread | | |

## Claims

1. An active retractor, comprising:
a retracting shaft (100) configured to fasten fractured bone fragments of a patient and retract the fractured bone fragments in a front and rear direction;
a measuring device (200) connected and installed to the retracting shaft (100) to measure a retracting location or a retracting force of the retracting shaft (100);
an actuator (300) connected and installed to the retracting shaft (100) to drive the retracting shaft in the front and rear direction;
a driver (400) configured to supply a power required for driving the actuator (300) to the actuator according to a driving control signal; and
a controller (500) configured to generate a driving control signal, which is required for the driver (400) to supply a power, by comparing a retracting request signal input from a user with a measured value transmitted from the measuring device (200) and transmit the driving control signal to the driver (400),
**characterized in that** the active retractor (10) further comprises:
a retracting arm (800) mounted and installed to the actuator (300) to operate in the front and rear direction, one side of the retracting shaft (100) being inserted in and fastened to the retracting arm (800) so that the retracting shaft (100) is driven in the front and rear direction by the retracting arm (800), the retracting arm (800) releasing the fastening of the retracting shaft (100) when a force equal to or greater than a preset force is applied thereto so that the retracting shaft (100) is released.

2. The active retractor according to claim 1,
wherein the measuring device (200) includes:
a retracting force measuring sensor (210) connected and installed to the retracting shaft (100) to measure a tensile force or a compressive force of the retracting shaft and then generate tensile force information or compressive force information corresponding thereto to be transmitted to the controller (500); and
a displacement measuring sensor (220) connected and installed to the retracting shaft (100) to measure a moved location of the retracting shaft and generate location information corresponding thereto to be transmitted to the controller (500).

3. The active retractor according to claim 2,
wherein the controller (500) includes:
a user interface unit (510) configured to display number information and graphic information required for manipulation and receive the retracting request signal from the user; and
a driving control unit (520) configured to generate the driving control signal, which is required for the driver to supply a power, by comparing the retracting request signal received from the user interface unit (510) with the measured value transmitted from the measuring device (200) and transmit the driving control signal to the driver (400).

4. The active retractor according to claim 3,
wherein the user interface unit (510) receives a retracting force, a retracting speed, a retracting location, a retracting start/stop, an emergency stop, or postural adjustment of a retracting instrument.

5. The active retractor according to claim 3,
wherein the user interface unit (510) receives from the user a retracting distance mode in which a bone fragment is retracted to a retracting location set by the user and then the retracted bone fragment is fixed or a retracting force mode in which a bone fragment is retracted while constantly maintaining a stress of the bone fragment to a retracting force set by the user.

6. The active retractor according to claim 3,
wherein the driving control unit (520) generates retracting force information corresponding to the tensile force information or the compressive force information transmitted from the retracting force measuring sensor (210) and transmits the retracting force information to the user interface unit (510).

7. The active retractor according to claim 3,
wherein the driving control unit (520) generates current location information or speed information of the retracting shaft (100) corresponding to the location information transmitted from the displacement measuring sensor (220) and transmits the current location information or speed information to the user interface unit (510).

8. The active retractor according to claim 3,
wherein the driving control signal is a control signal about a retracting speed, a retracting location or a retracting force of the retracting shaft in a digital or analog form.

9. The active retractor according to claim 1, further comprising:
a foothold switch (700) or a remote controller (600) located within a work area of the user to receive a retracting request signal or an operation mode from the user and transmit the retracting request signal or the operation mode to the controller (500).

10. The active retractor according to claim 1, further comprising:
a location display device (310) formed at one side of the actuator (300) to display a driving location of the retracting shaft (100) in real time.

11. The active retractor according to claim 10, further comprising:
a laser guide (320) formed at one side of the actuator (300) to guide a retracting direction and an aligning direction of a fractured area of a patient.

## Patentansprüche

1. Aktiver Retraktor, umfassend:
einen Retraktionsschaft (100), der konfiguriert ist, um gebrochene Knochenfragmente eines Patienten zu befestigen und die gebrochenen Knochenfragmente in einer Vorne-und-Hinten-Richtung zurückzuziehen;
eine Messvorrichtung (200), die mit dem Retraktionsschaft (100) verbunden und daran installiert ist, um einen Retraktionsort oder eine Retraktionskraft des Retraktionsschafts (100) zu messen;
einen Aktuator (300), der mit dem Retraktionsschaft (100) verbunden und daran installiert ist, um den Retraktionsschaft in der Vorne-und-Hinten-Richtung anzutreiben;
einen Treiber (400), der konfiguriert ist, um den Aktuator gemäß einem Treibersteuersignal Energie zuzuführen, die zum Antrieb des Aktuators (300) erforderlich ist; und
ein Steuergerät (500), das konfiguriert ist, um, durch Vergleich eines von einem Benutzer eingegebenen Retraktionsanforderungssignals mit einem von der Messvorrichtung (200) gemessenen Messwert ein Treibersteuersignal zu erzeugen, das der Treiber (400) benötigt, um Energie zuzuführen, und das Treibersteuersignal zu dem Treiber (400) zu senden,
**dadurch gekennzeichnet, dass** der aktive Retraktor (10) ferner aufweist:
einen Retraktionsarm (800), der an dem Aktuator (300) angebracht und installiert ist, um in der Vorne-und-Hinten-Richtung zu arbeiten, wobei eine Seite des Retraktionsschafts (100) in den Retraktionsarm (800) eingesetzt und dort befestigt ist, sodass der Retraktionsschaft (100) durch den Retraktionsarm (800) in der Vorne-und-Hinten-Richtung angetrieben wird, wobei der Retraktionsarm (800) die Befestigung des Retraktionsschafts (100) löst, wenn daran eine Kraft gleich oder größer als eine voreingestellte Kraft angelegt wird, sodass der Retraktionsschaft (100) gelöst wird.

2. Der aktive Retraktor nach Anspruch 1,
wobei die Messvorrichtung (200) enthält:
einen Retraktionskraftmesssensor (210), der mit dem Retraktionsschaft (100) verbunden und daran installiert ist, um eine Zugkraft oder eine Kompressionskraft des Retraktionsschafts zu messen, und dann dementsprechende Zugkraftinformation oder Kompressionskraftinformation zu erzeugen, die zum Steuergerät (500) zu senden ist; und
einen Hubbmesssensor (220), der mit dem Retraktionsschaft (100) verbunden und daran installiert ist, um einen bewegten Ort des Retraktionsschafts zu messen und um dementsprechende Ortsinformation zu erzeugen, die zu dem Steuergerät (500) zu senden ist.

3. Der aktive Retraktor nach Anspruch 2,
wobei das Steuergerät (500) enthält:
eine Benutzerschnittstelleneinheit (510), die konfiguriert ist, um Zahleninformation und graphische Information anzuzeigen, die zur Handhabung erforderlich ist, und um das Retraktionsanforderungssignal vom Benutzer zu empfangen; und
eine Treibersteuereinheit (520), die konfiguriert ist, um, durch Vergleich des von der Benutzerschnittstelleneinheit (510) empfangenen Retraktionsanforderungssignals mit dem von der Messvorrichtung (200) gesendeten Messwert, das Treibersteuersignal zu erzeugen, das der Treiber zur Energieversorgung benötigt, und das Treibersteuersignal zum Treiber (400) zu senden.

4. Der aktive Retraktor nach Anspruch 3,
wobei die Benutzerschnittstelleneinheit (510) eine Retraktionskraft, eine Retraktionsgeschwindigkeit, einen Retraktionsort, einen Retraktionsstart/stopp, einen Notstopp, oder eine Lageeinstellung eines Retraktionsinstruments empfängt.

5. Der aktive Retraktor nach Anspruch 3,
wobei die Benutzerschnittstelleneinheit (510) vom Benutzer einen Retraktionsdistanzmodus empfängt, in dem ein Knochenfragment zu einem vom Benutzer gesetzten Retraktionsort zurückgezogen wird, und dann das zurückgezogene Knochenfragment fixiert wird, oder einem Retraktionskraftmodus, in dem ein Knochenfragment zurückgezogen wird, während eine Belastung des Knochenfragments konstant auf einer vom Benutzer gesetzten Retraktionskraft gehalten wird.

6. Der aktive Retraktor nach Anspruch 3,
wobei die Treibersteuereinheit (520) Retraktionskraftinformation erzeugt, die der vom Retraktionskraftmesssensor (210) gesendeten Zugkraftinformation oder Kompressionskraftinformation entspricht, und die Retraktionskraftinformation zur Benutzerschnittstelleneinheit (510) sendet.

7. Der aktive Retraktor nach Anspruch 3,
wobei die Treibersteuereinheit (520) gegenwärtige Ortsinformation oder Geschwindigkeitsinformation des Retraktionsschafts (100) entsprechend der vom Hubmesssensor (220) gesendeten Ortsinformation erzeugt und die gegenwärtige Ortsinformation oder Geschwindigkeitsinformation zur Benutzerschnittstelleneinheit (510) sendet.

8. Der aktive Retraktor nach Anspruch 3,
wobei das Treibersteuersignal ein Steuersignal über eine Retraktionsgeschwindigkeit, einen Retraktionsort oder eine Retraktionskraft des Retraktionsschafts in digitaler oder analoger Form ist.

9. Der aktive Retraktor nach Anspruch 1, der ferner umfasst:
einen Fußhalteschalter (700) oder eine Fernsteuerung (600), der oder die in einem Arbeitsbereich des Benutzers angeordnet ist, um ein Retraktionsanforderungssignal oder einen Betriebsmodus von dem Benutzer zu empfangen und das Retraktionsanforderungssignal oder den Betriebsmodus zu dem Steuergerät (500) zu senden.

10. Der aktive Retraktor nach Anspruch 1, der ferner umfasst:
eine Ortsanzeigevorrichtung (310), die an einer Seite des Aktuators (300) ausgebildet ist, um einen Antriebsort des Retraktionsschafts (100) in Echtzeit anzuzeigen.

11. Der aktive Retraktor nach Anspruch 10, der ferner umfasst:
eine Laserführung (320), die an einer Seite des Aktuators (300) ausgebildet ist, um eine Retraktionsrichtung und eine Ausricht-Richtung eines gebrochenen Gebiets eines Patienten zu führen.

## Revendications

1. Rétracteur actif, comprenant :
un arbre de rétraction (100) configuré pour fixer des fragments d'os fracturé d'un patient et rétracter les fragments d'os fracturé dans une direction avant et arrière ;
un dispositif de mesure (200) raccordé à et installé sur l'arbre de rétraction (100) pour mesurer un emplacement de rétraction ou une force de rétraction de l'arbre de rétraction (100) ;
un actionneur (300) raccordé à et installé sur l'arbre de rétraction (100) pour entraîner l'arbre de rétraction dans la direction avant et arrière ;
un dispositif d'entraînement (400) configuré pour fournir une puissance requise pour l'entraînement de l'actionneur (300) à l'actionneur conformément à un signal de commande d'entraînement ; et
un dispositif de commande (500) configuré pour générer un signal de commande d'entraînement, qui est requis pour que le dispositif d'entraînement (400) fournisse une puissance, par comparaison d'un signal de demande de rétraction entré par un utilisateur à une valeur mesurée transmise par le dispositif de mesure (200) et transmettre le signal de commande d'entraînement au dispositif d'entraînement (400), **caractérisé en ce que** le rétracteur actif (10) comprend en outre :
un bras de rétraction (800) monté et installé sur l'actionneur (300) pour fonctionner dans une direction avant et arrière, un côté de l'arbre de rétraction (100) étant inséré dans et fixé au bras de rétraction (800) de manière à ce que l'arbre de rétraction (100) soit entraîné dans la direction avant et arrière par le bras de rétraction (800), le bras de rétraction (800) libérant la fixation de l'arbre de rétraction (100) lorsqu'une force supérieure ou égale à une force prédéfinie est appliquée à celui-ci de manière à ce que l'arbre de rétraction (100) soit libéré.

2. Rétracteur actif selon la revendication 1,
dans lequel le dispositif de mesure (200) inclut :
un capteur de mesure de force de rétraction (210) raccordé à et installé sur l'arbre de rétraction (100) pour mesurer une force de traction ou une force de compression de l'arbre de rétraction puis générer des informations de force de traction ou des informations de force de compression correspondant à celui-ci à transmettre au dispositif de commande (500) ; et
un capteur de mesure de déplacement (220) raccordé à et installé sur l'arbre de rétraction (100) pour mesurer un emplacement déplacé de l'arbre de rétraction et générer des informations de localisation correspondant à celui-ci à transmettre au dispositif de commande (500).

3. Rétracteur actif selon la revendication 2,
dans lequel le dispositif de commande (500) inclut :
une unité interface utilisateur (510) configurée pour afficher des informations numériques et des informations graphiques requises pour la manipulation et pour recevoir le signal de demande de rétraction de l'utilisateur ; et
une unité de commande d'entraînement (520) configurée pour générer le signal de commande d'entraînement, qui est requis pour que le dispositif d'entraînement fournisse une puissance, par comparaison du signal de demande de rétraction reçu de l'unité interface utilisateur (510) à la valeur mesurée transmise par le dispositif de mesure (200) et transmettre le signal de commande d'entraînement au dispositif d'entraînement (400).

4. Rétracteur actif selon la revendication 3,
dans lequel l'unité interface utilisateur (510) reçoit une force de rétraction, une vitesse de rétraction, un emplacement de rétraction un début/une fin de rétraction, un arrêt d'urgence, ou un ajustement de posture d'un instrument de rétraction.

5. Rétracteur actif selon la revendication 3,
dans lequel l'unité interface utilisateur (510) reçoit de l'utilisateur un mode distance de rétraction dans lequel un fragment d'os est rétracté jusqu'à une position de rétraction définie par l'utilisateur puis le fragment d'os rétracté est fixé ou un mode force de rétraction dans lequel un fragment d'os est rétracté tout en maintenant constamment une contrainte du fragment d'os sur une force de rétraction définie par l'utilisateur.

6. Rétracteur actif selon la revendication 3,
dans lequel l'unité de commande d'entraînement (520) génère des informations de force de rétraction correspondant aux informations de force de traction ou aux informations de force de compression transmises par le capteur de mesure de force de rétraction (210) et transmet les informations de force de rétraction à l'unité interface utilisateur (510).

7. Rétracteur actif selon la revendication 3,
dans lequel l'unité de commande (520) génère des informations de localisation actuelle ou des informations de vitesse de l'arbre de rétraction (100) correspondant aux informations de localisation transmises du capteur de mesure de déplacement (220) et transmet les informations de localisation actuelles ou les informations de vitesse à l'unité interface utilisateur (510).

8. Rétracteur actif selon la revendication 3,
dans lequel le signal de commande d'entraînement est un signal de commande relatif à une vitesse de rétraction, un emplacement de rétraction ou une force de rétraction de l'arbre de rétraction sous une forme numérique ou analogique.

9. Rétracteur actif selon la revendication 1, comprenant en outre :
une pédale de commutation (700) ou un dispositif de commande à distance (600) situé à l'intérieur d'une zone de travail de l'utilisateur pour recevoir un signal de demande de rétraction ou un mode de fonctionnement de l'utilisateur et transmettre le signal de demande de rétraction ou le mode de fonctionnement au dispositif de commande (500).

10. Rétracteur actif selon la revendication 1, comprenant en outre :
un dispositif d'affichage d'emplacement (310) formé d'un côté de l'actionneur (300) pour afficher un emplacement d'entraînement de l'arbre de rétraction (100) en temps réel.

11. Rétracteur actif selon la revendication 10, comprenant en outre :
un guide laser (320) formé sur un côté de l'actionneur (300) pour guider une direction de rétraction et une direction d'alignement d'une zone fracturée d'un patient.
